# EUROPÄISCHE PATENTSCHRIFT

(11) **EP 0 698 373 B1**
(45) Veröffentlichungstag und Bekanntmachung des Hinweises auf die Patenterteilung: **05.11.2003**
(21) Anmeldenummer: 95113341.2
(22) Anmeldetag: 24.08.1995
(51) Int. Cl.: A61B 17/12, A61B 17/00

(54) **Verschlussvorrichtung zum Verschliessen einer Gefässöffnung**
Occlusion device for closing an opening in a vessel
Dispositif d'occlusion d'une ouverture d'un vaisseau

(30) Priorität: 11.01.1995 DE 29500381 U; 24.08.1994 DE 9413645 U
(43) Veröffentlichungstag der Anmeldung: 28.02.1996
(73) Patentinhaber: Cardia, Inc., Burnsville, MN 55337 (US)
(72) Erfinder: Schneidt, Bernhard, 63571 Gelnhausen (DE); Köhler, Klaus Peter, 65611 Niederbrechen (DE); Schräder, Rainer Dr., 60596 Frankfurt (DE)
(74) Vertreter: Patentanwälte Hauck, Graalfs, Wehnert, Döring, Siemons, Schildberg

(56) Entgegenhaltungen:
- EP-A- 0 362 113
- EP-A- 0 541 063
- WO-A-93/16650
- WO-A-94/09705
- WO-A-94/09706
- DD-A- 233 303
- DE-C- 4 222 291
- DE-U- 9 413 645
- JP-B- 57 024 132
- US-A- 3 874 388
- US-A- 4 007 743
- US-A- 5 108 420
- US-A- 5 334 137

## Beschreibung

Die Erfindung bezieht sich auf eine Verschlußeinrichtung zum Verschließen einer körperlichen Anomalie wie Gefäßöffnung wie Ductus arteriosus, Atrium-septum-Defekt, Foramen ovale oder Ventricel-septum-Defekt, umfassend einen sich innerhalb der Gefäßöffnung erstreckenden Verschlußkörper sowie außerhalb der Gefäßöffnung erstreckende drahtförmige elastische Arretierungsmittel. Ferner bezieht sich die Erfindung auf eine Verschlußeinrichtung zum Verschließen einer körperlichen Anomalie wie Gefäßöffnung wie Ductus arteriosus, Atrium-septum-Defekt, Foramen ovale oder Ventricel-septum-Defekt, umfassend einen sich innerhalb der Gefäßöffnung erstreckenden Verschlußkörper mit axial sich erstreckendem Mittelteil.

Der menschliche Blutkreislauf besteht aus einem Herz- und einem Lungenkreislauf. In der embryonalen Phase der Entwicklung eines Menschen sind die beiden Kreisläufe durch den Ductus arteriosus miteinander verbunden. Der Ductus verbindet die Aorta (Körperkreislauf) mit der Pulmonalarterie (Lungenkreislauf). Bei einer normalen Entwicklung eines Säuglings verwächst dieser Ductus nach der Geburt. Bei krankhafter Entwicklung kann der Fall eintreten, daß der Ductus nicht zuwächst, wodurch die beiden Blutkreisläufe auch nach der Geburt miteinander verbunden bleiben. Dadurch kann die Lebenserwartung des Säuglings stark reduziert werden.

Es ist bekannt, den Ductus durch einen chirurgischen Eingriff zu verschließen. Dieser Eingriff ist jedoch sehr kostenintensiv und ist mit einem Risiko für den Patienten verbunden.

Weiterhin ist es bekannt, den Ductus mittels eines IVALON®-Schaumstoffpfropfens zu verschließen (Porstmann-Technik). Dabei wird eine Leitschiene durch eine Femoralarterie in die Aorta eingeführt, durch den Ductus in die Pulmonalarterie und von dort durch die rechte Herzkammer und den rechten Vorhof und schließlich über die gegenseitige Femoralvene wieder nach außen geführt. Anschließend wird der Ductuspfropfen in den Ductus geschoben, wo er "festgeklemmt" wird. Aufgrund des hohen Druckunterschiedes zwischen Aorta und Pulmonalarterie werden an die Arretierung des Ductuspfropfens innerhalb des Ductus hohe Anforderungen gestellt.

Daher müssen verhältnismäßig große Pfropfen verwendet werden, die in etwa 10-fach in der Länge gestaucht werden und 30% größer als der Durchmesser des Ductus sind. Entsprechend große Schleusen werden zur Einführung des Pfropfens in die Femoralarterie benötigt. Bei Säuglingen ist das Kaliber der Gefäße zu klein und oft bei Kindern < 30 kg auch nicht ausreichend für einen derartigen Eingriff.

Eine Verschlußeinrichtung der eingangs genannten Art ist der DD 233 303 A1 zu entnehmen. Bei der bekannten Verschlußeinrichtung weist der Verschlußkörper eine einschalige hyperboloidähnliche Grundform auf und kann aus Metall, einer Metallegierung, Kunststoff und ähnlichen Materialien bestehen. Außerhalb des Verschlußkörpers erstrecken sich drahtförmige Arretierungsmittel, die endseitig spitz auslaufen, so daß die Gefahr von Verletzungen von Gefäßwandungen besteht.

Zum Implantieren der Verschlußeinrichtung wird diese von einem sich innerhalb einer Sicherungshülse erstreckendem Haltedraht erfaßt und durch einen Katheter verschoben. Aufgrund der Form des Verschlußkörpers und der spitz zulaufenden Enden der Arretierungsmittel besteht mit erheblicher Verletzungsgefahr für einen Patienten die Möglichkeit, eine fehlgesetzte Verschlußeinrichtung wieder in den Katheter zurückzuziehen.

Aus WO 94/09 706 ist eine Verschlußvorrichtung mit einem schraubenförmigen Basiskörper bekannt, von dem Fasern ausgehen, die eine Thrombenbildung hervorrufen.

In DE 42 22 291 C1 wird eine Prothese zum Verschließen eines Atrial- oder eines Ventricular-Septal-Defektes beschrieben. Die Prothese umfaßt ein über eine Zugfeder vorgespanntes Mittelteil, von dem Arme ausgehen, die sich an eine Gefäßwand anlegen. Aus der US 3,874,388 ist eine Verschlußeinrichtung mit einem Grundkörper bekannt, von dessen Enden eine Schirmwirkung ausübende Arretierungsmittel ausgehen, die an Gefäßwänden anlegbar sind.

DE-U-94 13645 gehört zum Stand der Technik im Sinne des Artikels 54(2) EPÜ für die Ansprüche 4-7 und 9-11.

Der vorliegenden Erfindung liegt das Problem zugrunde, eine Verschlußeinrichtung der zuvor beschriebenen Art so weiterzubilden, daß die Arretierung der Verschlußeinrichtung innerhalb der Gefäßöffnung verbessert und somit ein sicherer Verschluß zur Verfügung gestellt wird. Auch soll die Implantation erleichtert werden, wobei die Möglichkeit gegeben sein soll, Fehlplazierungen ohne Schwierigkeiten zu korrigieren.

Das Problem wird erfindungsgemäß durch eine Vorrichtung mit den Merkmalen aus Anspruch 1 gelöst. Die Arretierungsmittel sind an ihren freien Enden kugel- oder linsenförmig ausgebildet oder mit kugel- oder linsenförmigen Elementen versehen. Durch die erfindungsgemäße Ausbildung der Verschlußeinrichtung ist sichergestellt, daß eine Verletzung der Gefäßwandungen durch die Arretierungsmittel ausgeschlossen wird. Hierdurch ergibt sich der Vorteil, daß beim Implantieren der Verschlußeinrichtung eine Lageveränderung des Verschlusses ohne weiteres möglich ist. Durch die kugel- bzw. linsenförmigen Enden der Arretierungsmittel ist auch gewährleistet, daß bei hohem Druckunterschied der zwischen den durch den Ductus verbundenen Gefäße die Fixierelemente, die normalerweise an den Innenwandungen der Gefäße anliegen, zu einer Verletzung aufgrund der Bewegung der Verschlußeinrichtung nicht führen können. Die kugeligen Enden verhindern auch eine Verletzung, selbst wenn die Vorrichtung gegebenenfalls ohne Sicherung des Katheters durch die Gefäße transportiert wird.

Die auch als Fixierelemente zu bezeichnenden Arretierungselemente gehen dabei strahlenförmig von dem Verschlußkörper aus. Alternativ besteht die Möglichkeit, die Arretierungsmittel als Schleifen auszubilden, wodurch sich größere Auflageflächen für die Arretierungsmittel an den Innenwandungen der Gefäße ergeben.

Die eine Funktion von Federelementen ausübenden Arretierungselemente können aus Platin, Nickel-Legierungen, Titan oder anderen geeigneten Materialien bestehen. Auch kann ein Nickeldraht mit Platinbeschichtung zum Einsatz gelangen. Die kugel- oder linsenförmigen Enden sich vorzugsweise polierte Titankugeln bzw. Laserschweißperlen.

Nach einer besonders hervorzuhebenden Ausgestaltung der Erfindung sind die Arretierungsmittel mit dem Verschlußkörper stirnflächenseitig durch Nähen lagefixiert. Hierdurch ist sichergestellt, daß der Verschlußkörper aufgrund des Druckunterschiedes an den Enden der Gefäßöffnung nicht verschoben bzw. kompremiert werden kann, sofern es sich um ein Kunststoffteil handeln sollte.

Die Arretierungsmittel können über ein sich axial innerhalb des Verschlußkörpers erstreckendes Mittelteil verbunden sein. Durch das Mittelteil kann sodann eine Verbindung zu den den jeweiligen Gefäßen zugeordneten Arretierungsmitteln erfolgen. Das Mittelteil selbst kann ein- oder mehrteilig wie teleskopartig ausgebildet sein. Aufnahmen für die Arretierungsmittel können als Durchbrechungen in den Endabschnitten der Mittelteile ausgebildet sein. Die Endabschnitte des Mittelteils können als separate Aufnahmen ausgebildet sein, die mit dem Mittelteil verbunden wie verschweißt sind.

Erfindungsgemäß sollte jeder Ductus so dicht und mit so viel Implantationsmasse wie möglich verschlossen werden. Hierdurch ist auch gewährleistet, daß der Raum im Führungskatheter stets optimal ausgefüllt ist, d.h. das Implantationsmaterial (IVALON®) kann nur mit einigem Kraftaufwand auf die Applikationszange verschoben werden. Um ein Lösen oder unkontrolliertes Verschieben des Implantats zu den Arretierungselementen auszuschließen, ist erfindungsgemäß vorgesehen, daß diese stets mit dem Implantat vernäht sind. Um eine optimale Füllung des Ductus zu erreichen, kann die Einrichtung sowohl ein Mittelteil aufweisen als auch ohne ein solches ausgebildet sein.

In einer weiteren Ausbildung der Erfindung weist zumindest ein Arretierungsmittel von dessen Mittenbereich ausgehend und/oder das Mittelteil in axialer Erstreckung des Verschlußkörpers eine Anformung wie Kupplung auf, die von einer Führungszange erfaßbar ist. Durch diese vorzugsweise kugelförmige Anformung ist die Möglichkeit gegeben, daß die Verschlußeinrichtung mittels einer flexiblen Führungszange innerhalb eines Führungskatheters leicht handbar ist.

Um nach dem Implantieren der Verschlußeinrichtung ein Lösen von der Führungszange erst dann vorzunehmen, wenn eine ordnungsgemäße Positionierung erfolgt ist bzw. um gegebenenfalls ein Zurückziehen der Verschlußeinrichtung in den Katheter zu ermöglichen, sieht eine besonders hervorzuhebende Ausgestaltung der Erfindung vor, daß zwischen der Verschlußeinrichtung und der Führungszange eine Sicherheitsverbindung wie -faden besteht.

Um eine besonders gute Abdichtung der Gefäßöffnung (Shuntverbindung) zu gewährleisten, ist weiterhin vorgesehen, daß der als Implantat zu bezeichnende Verschlußkörper rotationssymmetrisch und zumindest bereichsweise kegelstumpfförmig ausgebildet ist. Das Implantat wird derart eingesetzt, daß eine verjüngte Seite des Implantats in Richtung des Gefäßes mit geringerem Druck zeigt. Die Oberfläche mit vergrößertem Querschnitt zeigt in Richtung des Gefäßes mit erhöhtem Druck. Dadurch wird die Shuntverbindung keilartig abgedichtet. Auch ein Verrutschen des Implantats innerhalb der Shuntverbindung kann vermieden werden.

In Weiterbildung der Erfindung kann das Implantat einen zylinderförmigen Basisbereich und einen kegelstumpfförmigen Spitzenbereich aufweisen, wobei der kegelstumpfförmige Spitzenbereich eine Oberfläche hat, die entweder einem Kegelstumpfmantel oder einer konkaven Form folgt.

Um eine bevorzugte Keilform zu erzielen, weist der Basisbereich eine Basisfläche mit einem Durchmesser D1 auf, der zumindest 4 mm größer ist als ein mittlerer Durchmesser MD des Implantats. Der mittlere Durchmesser MD wird dabei in der Mitte der axialen Erstreckung des Implantats an dem kegelstumpfförmigen Spitzenbereich gemessen.

Weitere Einzelheiten, Vorteile und Merkmale der Erfindung ergeben sich nicht nur aus den Ansprüchen, den diesen zu entnehmenden Merkmalen - für sich und/oder in Kombination -, sondern auch aus der nachfolgenden Beschreibung von der Zeichnung zu entnehmenden bevorzugten Ausführungsbeispielen.

Es zeigen:
- Fig. 1: eine erste Ausführungsform einer Verschlußeinrichtung in perspektivischer Darstellung,
- Fig. 2: eine Verschlußeinrichtung in perspektivischer Darstellung, die zur Erläuterung der Erfindung dient,
- Fig. 3: die Verschlußeinrichtung gemäß Fig. 1 eingeführt in einen Führungskatheter,
- Fig. 4: die an ihrem Einsatzort eingesetzte Verschlußeinrichtung gemäß Fig. 1,
- Fig. 5: eine erste Ausführungsform eines Verschlußkörpers für einen VE in Schnittdarstellung,
- Fig. 6: eine zweite Ausführungsform eines Verschlußkörpers in Schnittdarstellung,
- Fig. 7: eine Einführungszange zum Einführen eines Verschlußeinrichtung durch einen Katheter,
- Fig. 8: eine über einen Sicherheitsfaden mit der Einführungszange verbundene Verschlußeinrichtung,
- Fig. 9: eine Detaildarstellung eines Mittelteils einer Verschlußeinrichtung,
- Fig. 10: eine weitere Ausführungsform eines Mittelteils einer Verschlußeinrichtung,
- Fig. 11: ein Gerüst einer Verschlußeinrichtung,
- Fig. 12: eine bevorzugte Ausführungsform eines Verschlußkörpers,
- Fig. 13: der Verschlußkörper nach Fig. 12 mit mit diesem verbundenen Arretierungsmitteln,
- Fig. 14: den Verschlußkörper nach Fig. 13 in Draufsicht und
- Fig. 15: den Verschlußkörper gemäß Fig. 13, positioniert in einem Führungskatheter.

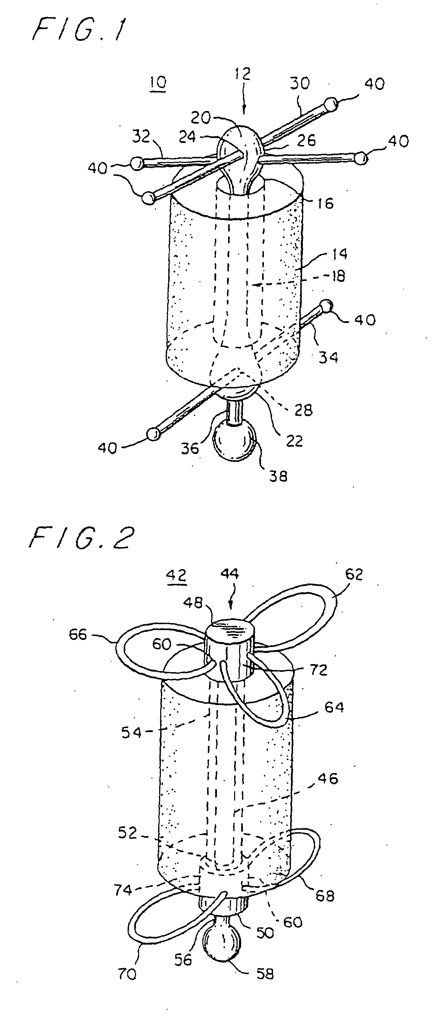

Fig. 1 zeigt eine erste Ausführungsform einer Verschlußeinrichtung (10), die - ohne daß die Erfindung hierdurch eingeschränkt werden soll -, als Ductusverschluß bezeichnet wird. Der Ductusverschluß (10) umfaßt eine Arretierungsvorrichtung (12) sowie ein vorzugsweise zylinderförmiges Implantat (14) als Verschlußkörper. Das Implantat (14) kann aus einem schaumstofförmigen Material wie IVALON® bestehen, das sich dadurch auszeichnet, daß es vom menschlichen Körper gut aufgenommen wird und mit dem umschließenden Gefäßgewebe eine Verbindung eingeht. Axial weist der eine Gefäßöffnung verschließende Verschlußkörper (14) eine Durchbrechung (16) auf, die zur Aufnahme der Arretierungsvorrichtung (12) ausgebildet ist. Selbstverständlich kann der Verschlußkörper (14) die Arretierungsvorrichtung (12) auch axial umschließen.

Die Arretierungsvorrichtung (12) weist ein Mittelteil (18) auf, daß in einer ersten Ausführungsform einteilig ausgebildet ist. Das Mittelteil (18) weist Endbereiche (20), (22) auf, in die Durchbrechungen (24), (26), (28) eingebracht sind, die zur Aufnahme von Arretierungsmitteln - auch Fixierelemente (30), (32), (34) genannt - dienen. Weiterhin weist das Mittelteil (18) an einem Endbereich (22) vorzugsweise durch einen Steg (36) oder eine Einschnürung beabstandet ein kugelförmiges oder zylinderförmiges Kupplungselement (38) auf. Das Mittelteil (18), der Steg (36) und die kugelförmige Kupplung (38) weisen eine gemeinsame Mittelachse auf.

Das Mittelteil (18) ist vorzugsweise aus einem Teil geschliffen oder gedreht, wobei ein Rundmaterial, z. B. aus Titan oder einem anderen Material wie Platinlegierung, mit einem Durchmesser von ca. 1 mm zur Erreichung einer hohen Flexibilität zwischen den Endbereichen (20), (22) auf einen Durchmesser von ca. 0,4 mm - 0,6 mm geschliffen oder gedreht wird. Die Endbereiche (20), (22) können eine kugelförmige oder zylindrische Form aufweisen. Die in den Endbereichen (20) eingebrachten Durchbrechungen (24), (26) verlaufen übereinander und sind vorzugsweise um einen Winkel von 90° zueinander versetzt.

Die Fixierelemente (30), (32), (34) können aus hochelastischem Titandraht bestehen, und weisen an ihren Enden polierte Laserschweißperlen (40) auf, um Verletzungen der Gefäßwände zu vermeiden. Die Enden können auch mit polierten Titankugeln versehen sein, die eine Sacklochbohrung von ca. 0,3 mm - 0,4 mm Durchmesser aufweisen und mit den Enden der drahtförmigen Fixierelemente (30), (32), (34) verschweißt sind.

Fig. 2 zeigt einen Ductusverschluß (22), der sich im wesentlichen durch eine geänderte Gestaltung einer Arretierungsvorrichtung (44) von der Ausführungsform gemäß Fig. 1 unterscheidet und lediglich zur Erläuterung der Erfindung dient. Die Arretierungsvorrichtung (44) weist ein Mittelteil (46) auf, an dessen Enden Halterungen (48), (50) befestigt wie verschweißt sind. Die Halterungen (48), (50) haben eine zylindrische Form und weisen Aufnahmen (52), (54) für das Mittelteil (47) auf. An der Halterung (50) ist an der der Aufnahme (52) gegenüberliegenden Seite ein Steg (56) angeformt, der in eine kugelförmige Kupplung (58) übergeht. Entsprechend Fig. 1 liegen das Mittelstück (47), der Steg (56) und die kugelförmige Kupplung (58) auf einer gemeinsamen Mittelachse.

Die Halterungen (48), (50) weisen weiterhin Aufnahmen (60) zur Aufnahme von Arretierungsmitteln bzw. Fixier- oder Halteelementen (62), (64), (66), (68), (70) auf. Die Aufnahmen (60) sind in Außenflächen (72), (74) der Halterungen (48), (50) auf einer Umfangslinie liegend eingebracht. Zur Bildung einer Schleife sind die Fixier- bzw. Halteelemente (62), (64), (66), (68), (70) jeweils mit ihren Enden in den Aufnahmen (60) der Halteelemente (48), (50) befestigt. Die Halte- bzw. Fixierelemente (62), (64), (66), (68), (70), die vorzugsweise aus hochelastischem Titandraht bestehen, werden in den Aufnahmen (60) mechanisch fixiert. Durch die bevorzugte schleifenförmige Ausbildung der Fixier- bzw. Halteelemente (62), (64), (66), (68), (70) werden Verletzungen von Gefaßwänden vermieden. Auch wird dadurch eine möglichst große Auflagefläche an den Gefäßinnenwänden erreicht.

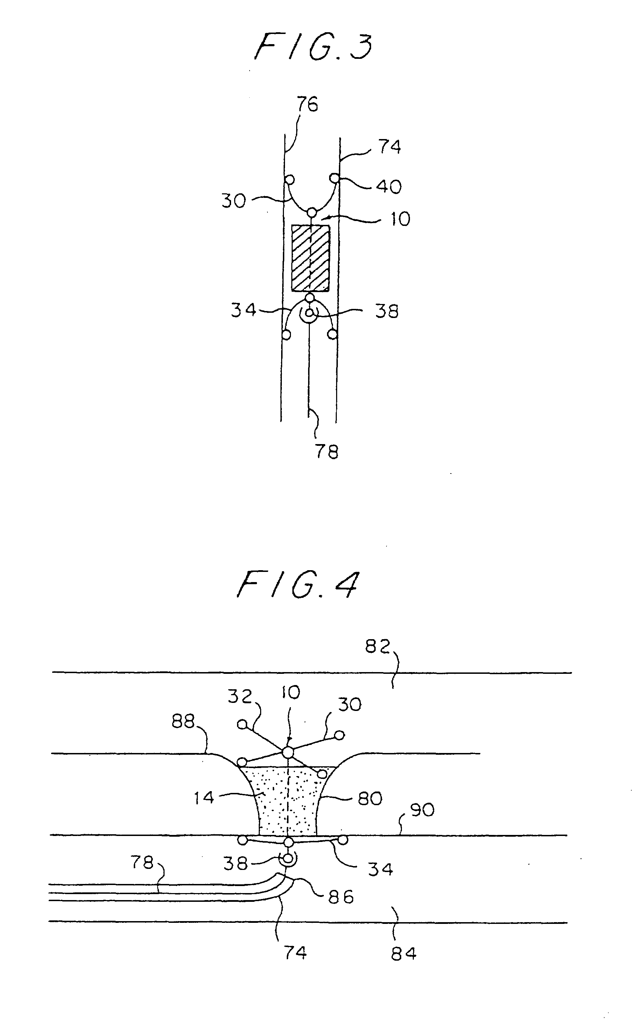

Fig. 3 zeigt den in einem Führungskatheter (74) eingelegten Ductusverschluß (10) gemäß Fig. 1. Der Ductusverschluß (10) liegt dabei mit seinen gebogenen Fixierelementen (30), (32), (34) sowie den polierten Laserschweißperlen oder Titankugeln (40) an einer Innenwandung (76) des Katheters (74) an. Durch das punktförmige Anliegen des Ductusverschlusses (10) innerhalb des Katheters (74) wird ein leichtes Bewegen des Ductusverschlusses (10) ermöglicht. Im ursprünglichen Zustand läßt sich das zylinderförmige Dichtungsimplantat wie IVALON® zusammen mit der Arretierungsvorrichtung (12), (44) leicht in den Katheter (74) einführen. Innerhalb des Katheters wird der Ductusverschluß (10) durch eine flexible Führungszange (78), die an der kugel- beziehungsweise zylinderförmigen Kupplung (38) des Ductusverschlusses (10) eingreift, gehalten.

Fig. 4 zeigt den in einen Ductus (80) eingebrachten Ductusverschuß (10). Der Ductusverschluß (10) wird über den Katheter (74) zum Beispiel von einer Beinvene über untere Hohlvene, rechten Vorhof, rechte Kammer und Pulmonalarterie (84) in den Ductus (80) eingebracht. Der Katheter (74) bzw. die Führungszange (78) weisen Durchmesser auf, die im Bereich von 2 mm bis 6 mm liegen. Insbesondere bei kleinen Durchmessern, wie z.B. 2,5 mm, besteht die Möglichkeit, auch Säuglinge und Kleinkinder zu behandeln.

Der Ductus (80) bildet hier die Verbindung zwischen einem Gefäß (82) wie Aorta und einem weiteren Gefäß (84) wie Lungenarterie. In der wie in Fig. 3 gezeigten Anordnung wird der Ductusverschluß (10) mittels des Katheters (74) in den Ductus (80) eingesetzt. Dazu wird der Katheter (74) mit eingelegtem Ductusverschluß (10) durch die Lungenarterie (84) in den Ductus (80) eingeführt, bis eine Oberkante (86) des Katheters (74) in die Aorta (82) hineinragt. Der Ductusverschluß (10) liegt dabei in Höhe des Ductus (80). Anschließend wird die Wandung (76) des Katheters (74) bei konstant gehaltener Position des Ductusverschlusses (10) in Richtung zur Arterie (84) bewegt, wodurch sich die Fixierelemente (30), (32) an einer Innenwandung (88) der Aorta (82) anlegen können. Anschließend wird das Implantat (14) freigelegt, so daß es sich an die Innenwandung (90) des Ductus (80) anpassen kann. Die Position des Ductusverschlusses (10) wird dabei durch die Führungszange (78), die an der Kupplung (38) des Ductusverschlusses (10) angreift, konstant gehalten. Nachdem das Implantant (14) freigelegt ist, wird schließlich das der Lungenarterie (84) zugewandte Fixierelement (34) freigelegt, das sich somit an die Innenwandung (92) der Lungenarterie (84) anpassen kann.

Durch den Kontakt mit Flüssigkeit wie Blut quillt das Implantat (14) auf, so daß der Ductus (80) dicht verschlossen wird. Durch die an den Innenwandungen (88), (92) anliegenden Fixierelemente (30), (32), (34) wird eine axiale Verschiebung des Implantats (14) aufgrund des hohen Druckunterschiedes zwischen den Gefäßen (82, 84) verhindert. Zur weiteren Fixierung des Implantats ist vorgesehen, daß das Implantat (14) mit der Arretierungsvorrichtung (12) mittels Nahtmaterial vernäht wird. Dadurch wird eine Relativbewegung zwischen Implantat (14) und Arretierungsvorrichtung (12) unterbunden. Nachdem der Ductusverschluß innerhalb des Ductus (80) eingesetzt ist, und die Fixierelemente (30), (32), (34) an den Innenwandungen (88), (92) der Gefäße (82), (84) anliegen, kann die flexible Führungszange (78) von der kugelförmigen Kupplung (38) gelöst werden und aus der Lungenarterie (84) entfernt werden. Die Geometrie des Implantats (14) ist derart ausgelegt, daß die Endbereiche (20), (22) und die Fixierelemente (30), (32), (34) wenigstens teilweise von dem aufgequollenen Implantatmaterial eingefaßt werden. Durch die erfindungsgemäße Vorrichtung kann somit auf einfache und kostengünstige Weise ein sicherer Ductusverschluß realisiert werden.

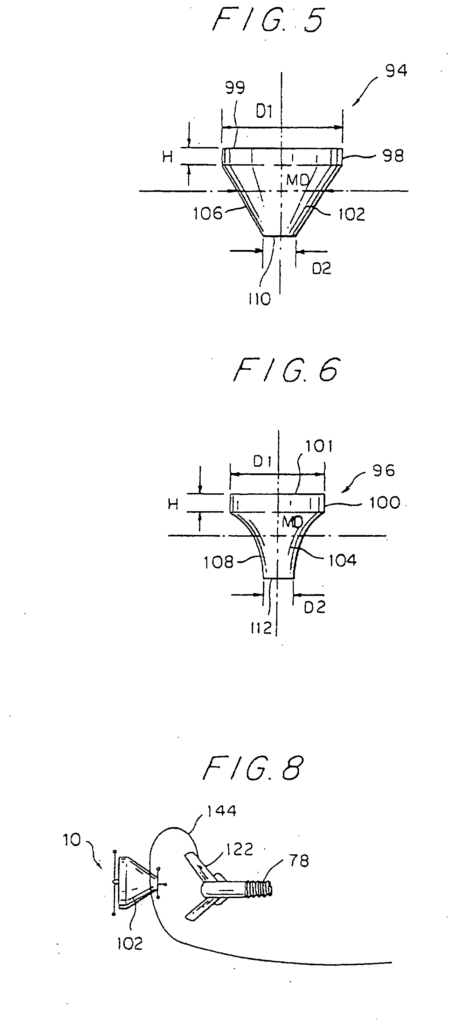

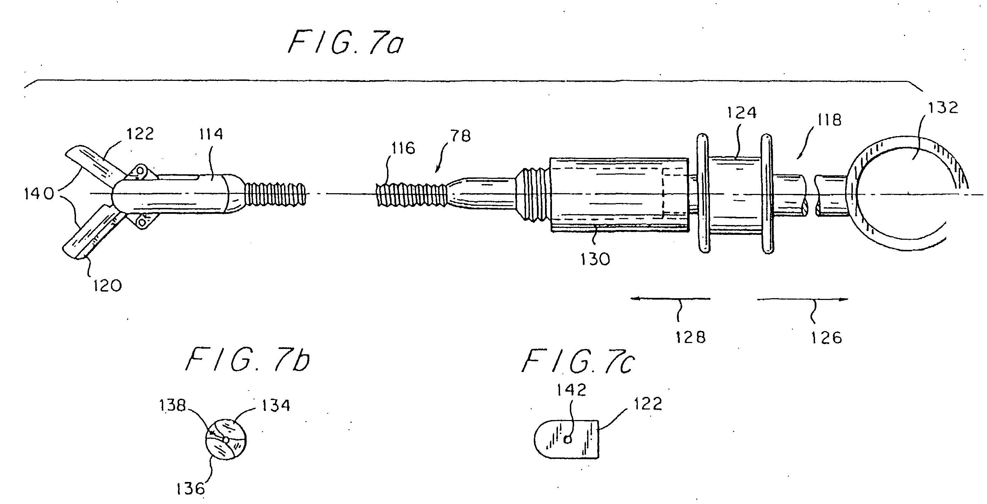

Die Fig. 5 und 6 zeigen verschiedene Ausführungsformen von Implantaten (94), (96). Bei den Implantaten (94), (96) handelt es sich um rotationssymmetrische Körper, die zumindest bereichsweise kegelstumpfförmig ausgebildet sind und vorzugsweise aus einem Schaumstoff wie Ivalon® bestehen.

Die Implantate (94), (96) bestehen im wesentlichen aus einem zylinderförmigen Basisbereich (98), (100), an den sich ein kegelstumpfförmiger Spitzenbereich (102), (104) anschließt. Der zylinderförmige Basisbereich (98), (100) weist einen Durchmesser D1 im Bereich von zum Beispiel 12 bis 14 mm auf, der jedoch vorzugsweise zumindest 4 mm größer ist als ein mittlerer Durchmesser MD des Implantats (94), (96). Als mittlerer Durchmesser MD wird ein Durchmesser des kegelstumpfförmigen Spitzenbereichs (102), (104) definiert, der in der Mitte zwischen Basisfläche (99), (101) und Endfläche (110), (112) ermittelt wird. Die Endfläche (110), (112) hat einen Durchmesser D2, der vorzugsweise im Bereich zwischen 4 bis 6 mm liegt. Ferner weist der zylinderförmige Basisbereich (98), (100) eine Höhe H auf, die vorzugsweise 2 mm beträgt.

Gemäß Fig. 5 schließt sich an den zylinderförmigen Basisbereich (98) ein kegelstumpfförmiger Spitzenbereich (102) an, dessen Oberfläche (106) der Form eines Kegelstumpfmantels folgt.

Alternativ kann der kegelstumpfförmige Spitzenbereich (104) gemäß Fig. 6 eine Oberfläche (108) aufweisen, die konkav verläuft.

In Fig. 7a ist die flexible Einführungszange (78) für den Ductus-Verschluß (10), (42) dargestellt. Die Einführungszange (78) weist einen Zangenkörper (114) auf, die über ein flexibles Zwischenstück (116) mit einer Handhabe (118) verbunden ist. Der Zangenkörper (114) besteht aus zwei schalenförmigen Klauenelementen (120), (122), die über ein Gestänge (nicht dargestellt), welches innerhalb des flexiblen Zwischenstücks (116) verläuft, mit einem Betätigungsring (124) der Handhabe (118) in Verbindung steht. Durch Verschieben des Betätigungsrings (124) in Richtung des Pfeils (126) können die schalenförmigen Klauenelemente (120), (122) verschlossen werden. Eine Bewegung des Betätigungsrings (124) in Richtung des Pfeils (128) hingegen bewirkt ein Öffnen der Klauenelemente (120), (122).

In geschlossener Stellung der Klauenelemente (120), (122) kann der Betätigungsring (124) durch eine Gewindehülse (130) arretiert werden. Zum Öffnen der Klauen (120), (122) ist somit ein Lösen der Gewindehülse (130) erforderlich, so daß der Betätigungsring (124) in Richtung des Pfeils (128) verschoben werden kann. Die Führungszange (78) selbst kann über einen Halterring (132) gehalten werden.

Fig. 7b zeigt eine Draufsicht in axialer Richtung auf die Klauenelemente (120), (122), wenn diese sich in geschlossener Stellung befinden. Die Klauenelemente (120), (122) weisen dabei jeweils Aussparungen (134), (136) auf, die bei geschlossener Klauenstellung eine vorzugsweise kreisförmige Öffnung (138) in axialer Richtung der Einführzange (78) bilden. Die Öffnung (138) dient zur Aufnahme des an das Arretierungsmittel (12), (44) angeformten Steges (36), (56), an den in axialer Erstreckung die Anformung (38), (58) wie Kupplung angeformt ist. In geschlossener Stellung der Klauen (120), (122) befindet sich die Anformung (38), (58) in einem durch die wannenförmigen Klauen ausgebildeten Hohlraum (140).

Fig. 7c zeigt eine Seitenansicht des Klauenelementes (122). Die Klauenwandung weist eine Durchbrechung (142) zur Aufnahme eines Sicherheitsfadens (144) auf, der zur Arretierung des Ductus-Verschlußes (10), (42) dient.

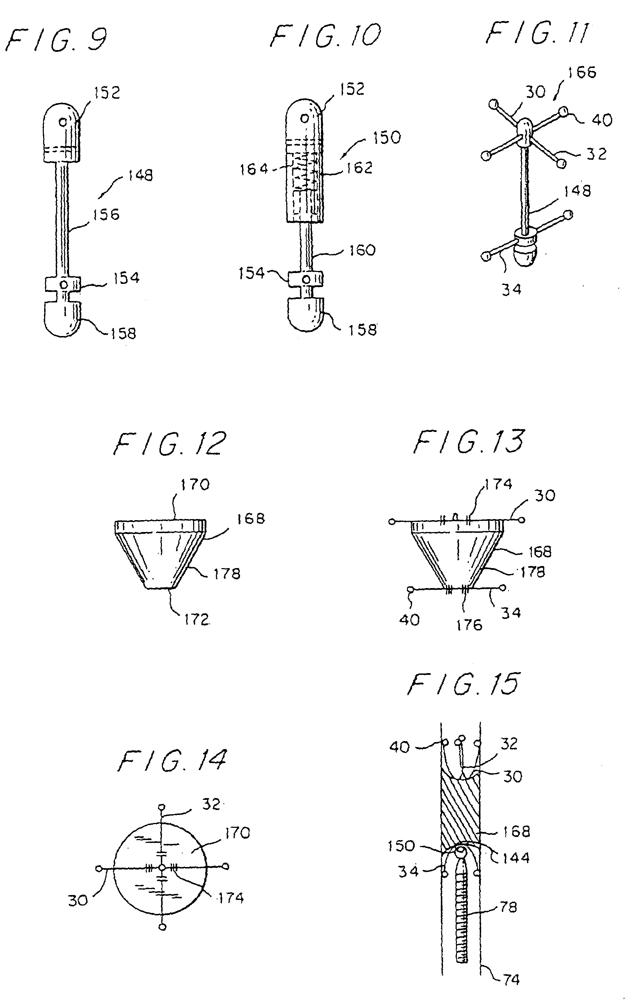

Fig. 8 zeigt die Sicherung des Ductus-Verschlußes (10) mittels des Sicherheitsfadens (144) an der Einführungszange (78). Dazu wird der Sicherheitsfaden (144) durch das Implantat (102) geführt und mit einem Ende (146) in der Öffnung (142) des Klauenelementes (122) verknotet. Durch den Sicherheitsfaden (144) ist der Ductus-Verschluß (10) auch dann gesichert, wenn die Klauenelemente (120), (122) unbeabsichtigt öffnen, oder wenn dieser nach gewolltem Abkoppeln verrutscht. Die Gefahr einer Embolie wird dadurch vermindert. Nach gelungener Implantation wird der Sicherheitsfaden (144) problemlos mit der Einführungszange (78) wieder durch den Führungskatheter gezogen.

In den Fig. 9 und 10 sind besondere Ausführungsformen von Mittelteilen (148) und (150) dargestellt, die sich in axialer Richtung eines Verschlußkörpers erstrecken und endseitige Verstärkungen (152), (154) aufweisen, die von nicht dargestellten Arretierungs- oder Fixiermitteln durchsetzt sind und mit diesen auf mechanische Weise verbunden werden. Zusätzlich sollten die Arretierungsmittel mit dem Verschlußkörper durch Dreifach- bzw. Doppelnaht mit drei Knoten vernäht werden, um eine Relativbewegung des Verschlußkörpers entlang des Mittelteils (148) und (150) zu unterbinden.

Das Mittelteil (148) erstreckt sich mit einem stegförmigen Mittelabschnitt (156), innerhalb eines nicht dargestellten Verschlußkörpers. Die querschnittvergrößerten Endbereiche (152) und (154) verlaufen zumindest im nicht implantierten Zustand der Verschlußeinrichtung außerhalb des Verschlußkörpers.

Endseitig, und zwar im Bereich des verstärkten Endes (154) weist jedes Mittelteil (148), (150) im Zusammenhang mit den Fig. 1, 2, 3 und 4 beschriebene und als Kupplung dienende Anformungen (158) auf, um das Mittelteil (148), (150) und damit die jeweilige Verschlußeinrichtung von einer Einführungszange zu erfassen.

Ist das Mittelteil (148) gemäß Fig. 9 einteilig ausgebildet, so weist die Ausführungsform nach Fig. 10 eine Längenveränderbarkeit dadurch auf, daß ein stegförmiger unterer Abschnitt (160) teleskopartig zum oberen und ersteren umgebenden Abschnitt (162) verstellbar ist. Dabei besteht zwischen dem äußeren Abschnitt (162) und dem stegförmigen inneren Abschnitt (160) vorzugsweise eine Verbindung über eine Zugfeder (164), wodurch sich eine optimale Anpassung an die Länge eines zu verschließenden Ductus ergibt.

In Fig. 11 ist eine auch als Gerüst zu bezeichnende Arretierungsvorrichtung (166) für eine Verschlußeinrichtung dargestellt, die aus einem zum Beispiel der Fig. 9 entsprechendem Mittelteil (148) sowie endseitig von diesem ausgehenden Arretierungs- bzw. Fixierelementen (30), (32), (34) mit endseitigen polierten Titankugeln (40) besteht.

Ein entsprechendes Gerüst (164) wird nunmehr von einem vorzugsweise aus IVALON® bestehenden und als Pfropfen zu bezeichnenden Verschlußkörper (168) umgeben, der dem der Fig. 5 entspricht, so daß auf die diesbezüglichen Ausführungen verwiesen werden.

Nachdem das Gerüst (166) von dem IVALON®-Pfropfen (168) umgeben ist, werden die Arretierungsmittel (30), (32) und (34) mit den Stirnflächen (170), (172) des Verschlußkörpers (168) verbunden. Dabei können die oberen und sternförmig zueinander verlaufenden Arretierungselemente (30), (32) mittels Doppelnaht mit drei Knoten, das einstrahlige Arretierungsmittel (34) mit der Stirnseite (172) mittels Dreifachnaht mit drei Knoten fixiert werden. Die entsprechenden Verbindungen sind rein prinzipiell durch die Bezugszeichen (174) (Doppelnaht mit drei Knoten) und (176) (Dreifachnaht mit drei Knoten) angedeutet.

Zu erwähnen ist noch, daß die bevorzugte Ausführungsform der den Figuren 11 bis 14 zu entnehmen Verschlußeinrichtung nicht zwingend das Mittelteil (148) aufweisen muß. Vielmehr können die Arretierungsmittel (30), (32), (34) allein durch Festnähen an dem Verschlußkörper (168) fixiert werden.

Eine dem Aufbau der Fig. 11 - 14 entsprechende Verschlußeinrichtung ist in Fig. 15 in einer in einem Führungskatheter (74) eingebrachten Position dargestellt. Man erkennt, daß der Pfropfen (168) an den Innenwandungen des Katheters (74) anliegt und daß die Arretierungselemente (30), (32) bzw. (34) mit ihren aus Titankugeln bestehenden Enden (40) an der Innenwandung des Führungskatheters (74) anliegen. Ferner ist die Verschlußeinrichtung mittels einer flexiblen Führungs- und Fixierzange (78) über die kugelförmige Anformung (158) erfaßt. Ferner ist die Verschlußeinrichtung mit der Fixierzange (78) über den Sicherheitsfaden (144) verbunden.

Durch die kegelstrumpförmige Form des Pfropfens (168) ergibt sich des weiteren der Vorteil, daß ein Zurückziehen der Verschlußeinrichtung in den Führungskatheter (74), nachdem die Verschlußeinrichtung aus diesem herausgebracht worden ist, ohne Verletzungsgefahr möglich ist, da sich dann die unteren Arretierungselemente (34) zunächst an den kegelstumpfförmigen Abschnitt (178) des Pfropfens (168) anlegen können, da zwischen diesem und dem Führungskatheter (74) ein Freiraum besteht; denn der Durchmesser der Stirnfläche (172) sollte kleiner als der Innendurchmesser des Führungskatheters (74) sein.

## Patentansprüche

1. Verschlußeinrichtung (10, 42) zum Verschließen einer körperlichen Anomalie, wie eine Gefäßöffnung (80), insbesondere Ductus arteriosus, Antrium-septum-Defekt, Foramenovale oder Ventricel-septum-Defekt mit einem sich zumindest innerhalb der Gefäßöffnung erstreckenden Verschlußkörper (14, 94, 168, 184), der eine Vielzahl von sich in radialer Richtung erstreckenden drahtförmigen, elastischen Arretierungsmittel (30, 32, 34) aufweist, die im eingesetzten Zustand aus der Gefäßöffnung vorstehen, **dadurch gekennzeichnet, daß** die Arretierungsmittel (30, 32, 34) an ihren freien Enden kugel- oder linsenförmig ausgebildet oder mit kugelförmigen oder linsenförmigen Elementen (40) versehen sind und daß die elastischen Arretierungsmittel (30, 32, 34) mit ihren radial nach innen weisenden Enden an einem Mittelteil (18, 46, 150, 182, 194) befestigt sind, das sich in axialer Richtung durch den Verschlußkörper (40, 46, 94, 96, 168) erstreckt und eine in axialer Richtung sich erstreckende Kupplung (38, 58, 158, 186) aufweist, die beim Implantieren der Verschlußeinrichtung (10, 42, 180, 188, 200) von einer innerhalb eines Katheters (72) sich erstreckenden Führungszange (74) erfaßbar ist.

2. Verschlußeinrichtung nach Anspruch 1, **dadurch gekennzeichnet, daß** die kugeloder linsenförmigen Elemente (40) polierte Laserschweißperlen oder polierte Titankugeln sind.

3. Verschlußeinrichtung nach Anspruch 1 oder 2, **dadurch gekennzeichnet, daß** die Arretierungsmittel (30, 32, 34) mit dem Verschlußkörper (94, 168) stirnflächenseitig durch Nähen lagefixiert sind.

4. Verschlußeinrichtung nach einem der vorangehenden Ansprüche, **dadurch gekennzeichnet, daß** der Verschlußkörper (94, 96, 168) rotationssymmetrisch und zumindest bereichsweise kegelstumpfförmig ausgebildet ist.

5. Verschlußeinrichtung nach einem der vorangehenden Ansprüche, **dadurch gekennzeichnet, daß** der Verschlußkörper (94, 96, 168) einen zylinderförmigen Basisabschnitt (98, 100) und einen kegelstumpfförmigen spitzen Abschnitt (102, 104, 178) aufweist, dessen Oberfläche (106) einem Kegelstumpfmantel oder einer konkaven Form folgt, wobei vorzugsweise der Basisabschnitt eine Basisfläche mit einem Durchmesser D1 aufweist, der zumindest 4mm größer als ein mittlerer Durchmesser MD gemessen auf der Hälfte der axialen Erstreckung des Verschlußkörpers ist.

6. Verschlußeinrichtung nach Anspruch 5, **dadurch gekennzeichnet, daß** von zumindest einem Arretierungsmittel (34) und/oder dem Verschlußkörper (94, 168) und/oder dem Mittelteil ein mit der Führungszange (74) verbindbares Sicherheitselement wie ein Sicherheitsfaden (144) ausgeht, das zur Verbindung mit der Führungszange (74) vorgesehen ist.

7. Verschlußeinrichtung nach einem der vorangehenden Ansprüche, **dadurch gekennzeichnet, daß** das Mittelteil (150) längenveränderbar ausgebildet ist und vorzugsweise aus zwei teleskopartig zueinander verschiebbaren Abschnitten (160, 162) besteht.

8. Verschlußeinrichtung nach einem der vorangehenden Ansprüche, **dadurch gekennzeichnet, daß** das Mittelteil (18, 46, 148, 150, 194) endseitig Verstärkungen (20, 22, 48, 50, 152, 196, 198) aufweist, an denen die Arretierungsmittel (30, 32, 24) befestigt sind.

9. Verschlußeinrichtung nach Anspruch 6, **dadurch gekennzeichnet, daß** der Verschlußkörper ausgebildet ist derart, daß dieser unter Verwendung der Führungszange (74), die einen Zangenkörper (114) und ein mit einer Handhabe (118) verbundenes flexibles Zwischenstück (116) aufweist, einsetzbar ist.

10. Verschlußeinrichtung nach Anspruch 6, **dadurch gekennzeichnet, daß** die Kupplung (38, 58, 158, 186) geformt ist derart, daß diese von zwei schalenförmigen Klauenelementen (120, 12) des Zangenkörpers (114) ergreifbar ist.

11. Verschlußeinrichtung nach einem der vorangehenden Ansprüche, **dadurch gekennzeichnet, daß** die Arretierungsmittel (30, 32, 34) vollständig oder nahezu vollständig innerhalb des Verschlußkörpers (202) verlaufen und vorzugsweise in dem Verschlußkörper eingeschäumt sind.

## Claims

1. An occluding device (10, 42) to occlude a corporeal anomaly such as a vascular hole (80), e.g. a ductus arteriosus, an atrium septum defect, a foramen ovale or a ventricle septum defect, comprising an occluding body (14, 94, 168, 184) sized to extend at least inside the vessel hole and said occluding body being provided with a plurality of radially projecting wire-shaped resilient arresting means (30, 32, 34) fixed to said occluding body and adapted in use to extend outside the vascular hole, **characterized in that** the arresting means (30, 32, 34) have their free ends formed with globular or lenticular shapes or are provided with globular or lenticular members (40), and said resilient arresting means (30, 32, 34) being fixed at radially inward ends to a middle part (18, 46, 150, 182, 194), which extends to the axial direction of the occluding body (40, 46, 94, 96, 168) and having a lug (38, 58, 158, 186) in an axial extension which lug is adapted to be gripped by a pair of pliers (74) extending within a catheter (72) while the occluding device is being implanted.

2. The occluding device according to claim 1, **characterized in that** the globular or lenticular members (40) are polished laser welding beads or polished titanium globules.

3. The occluding device according to claim 1 or 2, **characterized in that** the arresting means (30, 32 34) are fixed in position by having their front-end faces sewn to the occluding bodies (94, 168).

4. The occluding device according to one of the preceding claims, **characterized in that** the occluding body (94, 96, 168) is rotationally symmetric and is of a truncated shape at least in some areas.

5. The occluding device according to one of the preceding claims, **characterized in that** the occluding body (94, 96, 168) has a cylinder-shaped basic portion (98, 100) and a truncated, pointed portion (102, 104, 178) the surface (106) of which follows truncated-cone envelope or a concave shape with the basic portion preferably having a base surface of a diameter D1 which is at least by 4mm larger than a mean diameter MD as measured half-way on the axial extension of the occluding body.

6. The occluding device according to claim 5, **characterized in that** a safety member such as a safety thread (144) extends from at least one arresting means (34) and/or the occluding body (94, 168) and/or the central part, wherein the safety member is adapted to be joined to the pair of guide pliers (74).

7. The occluding device according to one of the preceding claims, **characterized in that** the central part (150) is designed to be variable in length and is preferably comprised of two portions (160, 162) which are telescopically displaceable towards each other.

8. The occluding device according to one of the preceding claims, **characterized in that** the central part (18, 46, 148, 150, 194) has its end sides fitted with reinforcing members (20, 22, 48, 50, 152, 158, 196, 198) to which the arresting means (30, 32, 34) are attached.

9. The occluding device according to claim 6, **characterized in that** the occluding device adapted such that it is implantable using the pair of guide pliers (74), the guide pliers (74) comprising a plier body (114) and a flexible intermediate piece (116) joined to a handle (118).

10. The occluding device according to claim 6, **characterized in that** the lug (38, 58, 158, 186) is adapted such that it can be gripped by two cup-shaped jaw members (120, 122) of the plier body (114).

11. The occluding device according to one of the preceding claims, **characterized in that** the resting means (30, 32, 34) extend completely or nearly completely within the occluding body and preferably are foamed into the occluding the body.

## Revendications

1. Dispositif d'obturation (10, 42) pour fermer une anomalie corporelle telle qu'une ouverture de vaisseau (80), en particulier le défaut ductus arteriosus, le défaut de communication interauriculaire, le défaut foramen ovale ou le défaut de communication interventriculaire, comprenant un corps d'obturation (14, 94, 168, 184) s'étendant au moins à l'intérieur de l'ouverture de vaisseau et comportant une pluralité de moyens d'arrêt (30, 32, 34) filiformes et élastiques, s'étendant radialement et débordant de l'ouverture du vaisseau à l'état inséré, **caractérisé en ce que** les moyens d'arrêt (30, 32, 34) présentent la forme de sphère ou de lentille à leurs extrémités libres, ou sont dotés d'éléments sphériques ou lenticulaires (40), et que les extrémités des moyens d'arrêt élastiques (30, 32, 34) dirigées radialement vers l'intérieur sont fixées a une pièce médiane (18, 46, 150, 182, 194) qui s'étend axialement à travers le corps d'obturation (40, 46, 94, 96, 168) et comporte un dispositif d'accouplement (38, 58, 158, 186) s'étendant axialement et pouvant être saisi lors de l'implantation du dispositif d'obturation (10, 42, 180, 188, 200) par une pince de guidage (78) s'étendant à l'intérieur d'un cathéter (72).

2. Dispositif d'obturation selon la revendication 1, **caractérisé en ce que** les éléments sphériques ou lenticulaires (40) sont des perles de soudures de laser polies ou des sphères de titane polies.

3. Dispositif d'obturation selon la revendication 1 ou 2, **caractérisé en ce que** les moyens d'arrêt (30, 32, 34) sont fixés dans leur position par couture du côté frontal sur le corps d'obturation (94, 168).

4. Dispositif d'obturation selon l'une des revendications précédentes, **caractérisé en ce que** le corps d'obturation (94, 96, 168) est symétrique par rotation et présente au moins une partie tronconique.

5. Dispositif d'obturation selon l'une des revendications précédentes, **caractérisé en ce que** le corps d'obturation (94, 96, 168) comporte une partie de base cylindrique (98, 100) et une partie de pointe tronconique (102, 104, 178) dont la surface latérale (106) suit la surface d'un cône tronqué ou d'une forme concave, la partie de base présentant de préférence une surface de base ayant un diamètre D1 qui est d'au moins 4 mm plus grand qu'un diamètre moyen MD, mesuré à la moitié de la longueur axiale du corps d'obturation.

6. Dispositif d'obturation selon la revendication 5, **caractérisé en ce qu'**un élément de sécurité, par exemple un fil de sécurité (144), prévu pour être raccordé à la pince de guidage (78) et pouvant être raccordé à la pince de guidage (78), s'étend à partir d'un moyen d'arrêt (34) au moins et/ou du corps d'obturation (94, 168) et/ou de la partie médiane.

7. Dispositif d'obturation selon l'une des revendications précédentes, **caractérisé en ce que** la partie médiane (150) est apte à changer de longueur et consiste de préférence en deux parties (160, 162) translatables l'une par rapport à l'autre de manière télescopique.

8. Dispositif d'obturation selon l'une des revendications précédentes, **caractérisé en ce que** la partie médiane (18, 46, 148, 150, 194) comporte à ses extrémités des renforcements (20, 22, 48, 50, 152, 196, 198) sur lesquels sont fixés les moyens d'arrêt (30, 32, 34).

9. Dispositif d'obturation selon la revendication 6, **caractérisé en ce que** le corps d'obturation est configuré de telle façon qu'il est insérable au moyen de la pince de guidage (78) qui comporte un corps de pince (114) et une pièce intermédiaire (116) flexible reliée à une manette (118).

10. Dispositif d'obturation selon la revendication 6, **caractérisé en ce que** le dispositif d'accouplement (38, 58, 158, 186) présente une forme telle qu'il peut être saisi par deux éléments de pince en forme de coque (120, 122) du corps de pince (114).

11. Dispositif d'obturation selon l'une des revendications précédentes, **caractérisé en ce que** les moyens d'arrêt (30, 32, 34) s'étendent totalement ou presque totalement à l'intérieur du corps d'obturation (202) et sont de préférence noyés dans le corps d'obturation par une mousse.
